# EUROPEAN PATENT APPLICATION

(11) **EP 4 296 372 A1**
(43) Date of publication of application: **27.12.2023**
(21) Application number: 22382590.2
(22) Date of filing: 22.06.2022
(51) Int. Cl.: C12Q 1/6804, C12Q 1/6869

(54) **METHOD TO DETECT AND DISCRIMINATE CYTOSINE MODIFICATIONS**

(71) Applicant: Universitat Pompeu Fabra (UPF), 08002 Barcelona (ES); Consejo Superior de Investigaciones Científicas (CSIC), 28006 Madrid (ES); Centre de Reglació Genómica (CRG), 08003 Barcelona (ES); Institució Catalana de Recerca i Estudis Avançats (ICREA), 08010 Barcelona (ES)
(72) Inventor: MARQUÈS BONET, Tomàs, 08002 Barcelona (ES); ESTELLER CUCALA, Paula, 28006 Barcelona (ES); LLOVERA NADAL, Laia, 28006 Madrid (ES); LIZANO GONZÁLEZ, Esther, 08002 Barcelona (ES)
(74) Representative: Cueto, Sénida

(57) **Abstract**

The present invention discloses a method to detect 5-hydroxymethylcytosine (5hmC) and discriminate between 5-methylcytosine (5mC) and 5-hydroxymethylcytosine (5hmC) from a nucleic acid that comprises enzymatic treatment and construction of nucleic acid library and a kit to perform said method.

## Description

### Field of the invention

The current invention relates to methods for analyzing methylation patterns in nucleic acids.

### Background

The analysis of Deoxyribonucleic acid (DNA) methylation by means of sodium bisulfite deamination of unmodified cytosines to uracils has been for many years the gold standard methodology. However, bisulfite sequencing alone cannot distinguish between different cytosine modifications such as 5-methylcytosine (5mC) and 5-hydroxymethylcytosine (5hmC). Sodium bisulfite modifies only unmethylated cytosines while 5mC and 5hmC are not converted. Other limitations of bisulfite sequencing include high levels of DNA fragmentation and GC bias. Therefore, high amounts of DNA and sequencing depth are needed.

The scientific article by Sun Z, Vaisvila R, Hussong LM, et al. Nondestructive enzymatic deamination enables single-molecule long-read amplicon sequencing for the determination of 5-methylcytosine and 5-hydroxymethylcytosine at single-base resolution. Genome Res. 2021;31(2):291-300. doi:10.1101/gr.265306.120 discloses a method to enzymatically differentiate between 5mC and 5hmC. However, a limitation of long-read amplicon sequencing technology employed is that this method is restricted to the analysis of a few genomic regions hindering the study of the whole methylome and hydroxymethylome of the organism studied.

The scientific article Kapp JD, Green RE, Shapiro B. A Fast and Efficient Single-stranded Genomic Library Preparation Method Optimized for Ancient DNA. J Hered. 2021;112(3):241-249. doi:10.1093/jhered/esab012 discloses a library-preparation method to sequence ancient DNA. This article does not provide any hint that would allow a skilled in the art to combine its teachings with any method to analyze DNA methylation patterns or to analyze the whole genome. The abstract of the article states [...] *convert natively single-stranded DNA and heat denatured double-stranded DNA into sequencing libraries* [...] This information would discourage a skilled in the art to use the findings of this document into a method that does not use heat to denature dsDNA as DNA is not naturally single-stranded.

The present invention discloses a method wherein the DNA is first enzymatically converted and then a whole genome sequencing library is prepared. The present invention not only solves the long-standing problem in the epigenetic field of sequencing 5mC and 5-hmC separately in whole genomes but also lowers the costs by eliminating the need for expensive DNA oligos.

### Description

The present invention discloses a method to detect 5-hydroxymethylcytosine (5hmC) in a nucleic acid sample, comprising the following steps:
**a)** providing a nucleic acid sample, said nucleic acid sample being
   a genomic DNA sample or
   a genomic DNA sample that has gone through a previous enrichment step for a target region of the genome,
   and fragmenting said nucleic acid sample into fragments of a sequence length between 100 base pairs and 10000 base pairs,
**b)** adding a protecting group to the 5-hydroxymethylcytosine (5hmC) in the fragmented nucleic acid sample of step a),
**d1)** denaturing the product of step b) to obtain a modified single stranded nucleic acid sample (ssNA),
**e1)** performing enzymatic deamination of the ssNA product obtained in step d1) to convert cytosines (C) and methylated cytosines (5mC) into uracils (U), and performing a cleanup of said deaminated ssNA,
**f1)** combining the products of step (e1) with
   a first adapter-splinted oligonucleotide complex, and
   a second adapter-splinted oligonucleotide complex
   by enzymatic ligation, wherein the first and second adapter-splinted oligonucleotide complex hybridize to the ssNA via a ssNA hybridization region,
**g1)** amplifying by indexing PCR the products of step f1) by combining said products with at least two sequencing adapters, to obtain a dsDNA nucleic acid library and
**h1)** sequencing the nucleic acid sample contained in the dsDNA nucleic acid library of step g1),
wherein the presence of a cytosine (C) in the same position of the dsDNA nucleic acid library and in the original nucleic acid sequence of step a) provides the location of 5hmC in a nucleic acid sample.

The method to detect 5-hydroxymethylcytosine (5hmC) in a nucleic acid sample is also named along the description as EOH-seq

In a particular embodiment, the percentages of (C) at each position provide a quantitative level of 5hmC at each location of the nucleic acid sample.

Another object of the present invention relates to a method to detect 5-methylcytosine (5mC) and 5-hydroxymethylcytosine (5hmC) in a nucleic acid sample comprising:
**a)** providing a nucleic acid sample, said nucleic acid sample being
   a genomic DNA sample or
   a genomic DNA sample that has gone through a previous enrichment step for a target region of the genome,
   and fragmenting said nucleic acid sample into fragments of a sequence length between 100 base pairs and 10000 base pairs,
**c)** adding a protecting group to the 5-hydroxymethylcytosine (5hmC) and oxidizing the 5-methylcytosine (5mC) of said fragmented second nucleic acid sample
**d2)** denaturing the product of step c) to obtain a modified single stranded nucleic acid sample (ssNA),
**e2)** performing enzymatic deamination of the ssNA product obtained in step d2) to convert cytosines (C) into uracils (U), and performing cleanup of said deaminated ssNA
**f2)** combining the products of step (e2) with
   a first adapter-splinted oligonucleotide complex, and
   a second adapter-splinted oligonucleotide complex
   by enzymatic ligation, wherein the first and second adapter-splinted oligonucleotide complex hybridize to the ssNA via a ssNA hybridization region,
**g2)** amplifying by indexing PCR the products of step f2) by combining said products with at least two different sequencing adapters, to obtain a dsDNA nucleic acid library and **h2)** sequencing the nucleic acid sample contained in the dsDNA nucleic acid library of step g2),
wherein the presence of a cytosine (C) in the same position of the dsDNA nucleic acid library and in the original nucleic acid sequence of step a) provides the location of 5hmC or 5mC in a nucleic acid sample.

The method to detect 5-methylcytosine (5mC) and 5-hydroxymethylcytosine (5hmC) in a nucleic acid sample is also named along the description as sEM-seq.

In a particular embodiment, the percentages of (C) at each position provide a quantitative level of 5hmC and 5mC at each location of the nucleic acid sample

Another object of the present invention relates to a method to detect and to discriminate 5-methylcytosine (5mC) from 5-hydroxymethylcytosine (5hmC) in a nucleic acid sample comprising:
providing a nucleic acid sample, said nucleic acid sample being
a genomic DNA sample or
a genomic DNA sample that has gone through a previous enrichment step for a target region of the genome,
fragmenting said nucleic acid sample into fragments of a sequence length between 100 base pairs and 10000 base pairs,
dividing it into at least a first nucleic acid sample and a second nucleic acid sample
   **1)** subjecting said fragmented first nucleic acid sample to steps b) to h1) of the above-mentioned EOH-seq method.
   **2)** subjecting said fragmented second nucleic acid sample to steps c) to h2) of the above-mentioned sEM-seq method:
   **3)** comparing the results of steps (1), (2), wherein the presence of a cytosine (C) in the sequence of the dsDNA nucleic acid library of either (1) and/or (2), and/or in the original nucleic acid sequence of step a) provides the location and discrimination of 5-methylcytosine (5mC) from 5-hydroxymethylcytosine (5hmC).

The method to detect and discriminate 5-methylcytosine (5mC) from 5-hydroxymethylcytosine (5hmC) in a nucleic acid sample is also named along the description as M-seq.

Step (1) is performed in a different tube from step (2). In a particular embodiment they can be performed at the same time or one after another.

In a particular embodiment the products of steps g1) and g2) are combined together for the sequencing of step h). Thus, step h) means both h1) and h2).

In a particular embodiment a genomic DNA sample comprises the whole genome of an organism.

In the present invention "whole genome" means that any region of the genome can be analyzed including autosomal and sexual chromosomes if present. There is no need to choose a particular target sequence from the genome to be analyzed or designing specific oligonucleotides for said target sequence.

Although the method of the invention is able to obtain the cytosine methylation and hydroxymethylation pattern of the whole genome, it may be possible that it is only obtained from some regions depending on the origin, amount and or quality of nucleic acid sample provided, such as few or single cells or free-flowing tumor or fetal nucleic acids.

In a particular embodiment, the nucleic acid sample comprises from 100 bp to 1×10⁶ bp of the whole genome of an organism after the previous enrichment step, preferably from 100 bp to 1×10⁷ bp, of the whole genome of an organism after the previous enrichment step, more preferably from 100 bp to 1×10⁸ bp, of the whole genome of an organism after the previous enrichment step, even more preferably from 100 bp to 1×10⁹ bp, of the whole genome of an organism after the previous enrichment step.

In a particular embodiment, the nucleic acid sample comprises about 0,0001% of the whole genome of an organism, preferably about 0,001% of the whole genome of an organism, more preferably about 0,01% of the whole genome of an organism, even more preferably about 0,1% of the whole genome of an organism, even more preferably about 1% of the whole genome of an organism, even more preferably about 5% of the whole genome of an organism, even more preferably about 10% of the whole genome of an organism, even more preferably about 20% of the whole genome of an organism, even more preferably about 30% of the whole genome of an organism, even more preferably about 40% of the whole genome of an organism, even more preferably about 50% of the whole genome of an organism, even more preferably about 60% of the whole genome of an organism, even more preferably about 60% of the whole genome of an organism, even more preferably about 70% of the whole genome of an organism, even more preferably about 80% of the whole genome of an organism, even more preferably about 90% of the whole genome of an organism, even more preferably about 100% of the whole genome of an organism.

The nucleic acid sample or DNA sample may result from an enrichment step, before step a) of the method of the invention including, but is not limited to antibody immunoprecipitation, chromatin immunoprecipitation, restriction enzyme digestion-based enrichment, hybridization-based enrichment, or chemical labeling-based enrichment.

An expert would know that the nucleic acid sample or a DNA sample must not go under any step that would alter the methylation and/or hydroxymethylation pattern such as a PCR reaction before subjecting said nucleic acid sample to the method of the invention.

In a particular embodiment the nucleic acid sample can be obtained from an organism from the Monera (bacteria), Protista, Fungi, Plantae, and Animalia Kingdoms. A nucleic acid sample can be obtained from a virus. Nucleic acid samples may be obtained from a patient or subject, from an environmental sample, or from an organism of interest. In embodiments, the nucleic acid sample is extracted or derived from a cell or collection of cells, a body fluid, a tissue sample, an organ, and an organelle. Preferably, the organism is an animal, for example a mammal, such as human.

The fragmentation of the nucleic acid sample can be done by any method known in the art, for example mechanical and enzyme-based methods, such as acoustic shearing, hydrodynamic shearing and nebulization, while enzyme-based methods include transposons, restriction enzymes and nicking enzymes. In a preferred embodiment the fragmentation is sonication, an example of acoustic shearing.

In a particular embodiment the nucleic acid samples are fragmented into fragments of a sequence length between 100 and 1000 bp, preferably between 200 and 800 bp, more preferably between 250 and 700 bp, even more preferably around 500 bp.

In another particular embodiment, the nucleic acid sample is in the range of 1 pg to about 1000 µg DNA, preferably from 10 pg to about 500 µg DNA, more preferably from 100 pg to about 100 µg DNA, even more preferably from 500 pg to about 10 µg DNA, even more preferably 1 ng to about 5 µg DNA, even more preferably from 50 ng DNA to 500 ng DNA.

The methods of the invention utilize mild enzymatic reactions that avoid the substantial degradation associated with methods like bisulfite sequencing. Thus, the methods of the present invention are useful in analysis of low-input samples, such as circulating cell-free DNA and in single-cell analysis.

In another particular embodiment of the invention, the nucleic acid sample comprises circulating cell-free DNA (cfDNA).

In addition, as the method of the invention can discriminate between 5hmC and 5mC in any region of the whole genome there is no need to design specific oligos to analyze target regions of the genome.

The nucleic acid sample used in the methods described herein may be from any source including, for example a body fluid, tissue sample, organ, organelle, or single cells. In embodiments, the DNA sample is circulating cell-free DNA (cell-free DNA or cfDNA), which is DNA found in the blood and is not present within a cell. cfDNA can be isolated from blood or plasma using methods known in the art. Commercial kits are available for isolation of cfDNA including, for example, the Circulating Nucleic Acid Kit (Qiagen). Approaches, reagents and kits for isolating, purifying and/or concentrating DNA from sources of interest are known in the art and commercially available. For example, kits for isolating DNA from a source of interest include the DNeasy^{®}, QIAamp^{®}, QIAprep^{®} and QIAquick^{®} nucleic acid isolation/purification kits by Qiagen, Inc. (Germantown, Mc); the DNAzol^{®}, ChargeSwitch^{®}, Purelink^{®}, GeneCatcher^{®} nucleic acid isolation/purification kits by Life Technologies, Inc. (Carlsbad, CA); the NucleoMag^{®}, NucleoSpin^{®}, and NucleoBond^{®} nucleic acid isolation/purification kits by Clontech Laboratories, Inc. (Mountain View, CA). In certain aspects, the nucleic acid is isolated from a fixed biological sample, e.g., formalin-fixed, paraffin-embedded (FFPE) tissue. Genomic DNA from FFPE tissue may be isolated using commercially available kits - such as the AllPrep^{®} DNA/RNA FFPE kit by Qiagen, Inc. (Germantown, Me), the RecoverAll^{®} Total Nucleic Acid Isolation kit for FFPE by Life Technologies, Inc. (Carlsbad, CA), and the NucleoSpin^{®} FFPE kits by Clontech Laboratories, Inc. (Mountain View, CA).

Both steps b) and c) add a protecting to the 5hmC in the nucleic acid sample so that it is not subject to deamination in the following step. For example, 5hmC in the nucleic acid sample can be rendered non-reactive to the subsequent steps by adding a protecting group to the 5hmC. In one embodiment, the protecting group is a sugar, including a modified sugar, for example glucose or 6-azide-glucose (6-azido-6-deoxy D-glucose).

The sugar protecting group can be added to the hydroxymethyl group of 5hmC by contacting the nucleic acid sample with uridine di phosphate (UDP)-sugar in the presence of a glucosyltransferase enzyme.

By stating that the protecting group is, for example, glucose, this refers to a glucose moiety (e.g., a beta-D-glucosyl residue) being added to 5hmC to yield glucosyl 5-hydroxymethyl cytosine (glu-5hmC). The sugar protecting group can be any sugar or modified sugar that is a substrate of the glucosyltransferase enzyme and prevents the subsequent deamination of the 5hmC.

In a particular embodiment, the step of adding a protecting group to the 5-hydroxymethylcytosine (5hmC) in the nucleic acid sample comprises contacting said nucleic acid sample with an enzyme, for example a β-glucosyltransferase (BGT).

In another particular embodiment BGT is the T4 Phage β-glucosyltransferase and/or the T4 Phage α-glucosyltransferase and derivatives or analogues thereof, for instance, an enzyme with a different aminoacid residue sequence that still retains about the same biological activity that BGT.

In step c), in addition of adding a protecting group to 5hmC, 5-methylcitosyne (5mC) in the nucleic acid sample is oxidized to be non-reactive to the subsequent steps.

In a particular embodiment, 5-methylcitosyne (5mC) in the nucleic acid sample is oxidized into 5-carboxylcytosine (5caC) and/or 5-Formylcytosine.

The oxidation of the nucleic acid part can be done by contacting the nucleic acid with an enzyme, for example a Tet Methylcytosine Dioxygenase (TET) enzyme.

In further embodiments, the TET enzyme is one or more of human TET1, TET2, and TET3 (Q8NFU7, Q6N021, 043151 UniProtKB Entry codes respectively); murine Tet1, Tet2, and Tet3 (Q3URK3, Q4JK59, Q8BG87 UniProtKB Entry codes respectively); *Naegleria* TET (NgTET) (D2W6T1, UniProtKB Entry code); and derivatives or analogues thereof.

In a particular embodiment there is a cleanup step after steps b) and c) and before step d1) and d2)

After protecting the 5mC and/or the 5hmC groups, the nucleic acid sample is denatured in step d1) and d2) into single stranded DNA (ssNA). A skilled person in the art would know how to perform this step by using common general knowledge.

In another particular embodiment it is used high temperature (from 70°C to 100°C, preferably from 80°C to 90°C) combined or not with:
sodium hydroxide or
formamide.

In a preferred embodiment, it is used high temperature, at a temperature comprised between 70°C to 100°C in the presence of formamide
"single-stranded nucleic acid" or "ssNA" is meant to refer to a collection of polynucleotides which are single-stranded (that is, not hybridized intermolecularly or intramolecularly) over 70% or more of their length. In some embodiments, the ssNA is single-stranded over 75% or more, 80% or more, 85% or more, 90% or more, 95% or more, or 99% or more, of the length of the polynucleotides. In certain aspects, the ssNA is single-stranded over the entire length of the polynucleotides.

In a particular embodiment, the deamination step is performed by an enzyme that deaminates cytidines, which comprises contacting the nucleic acid with an enzyme, for example a member of the Apolipoprotein B MRNA Editing Enzyme Catalytic (APOBEC gene family).

A further advantage of the method of the invention is that that the enzymatic-mediated deamination of steps e1) and e2) is less damaging for the nucleic acid sample compared to chemical treatments such as bisulfate treatment or borane reduction. In addition, bisulfate and borane are very toxic for human beings, thus the method of the invention also improves the safety of the users compared to the methods disclosed in the state of the art.

In further embodiments, the APOBEC enzyme is one or more of human APOBEC3G, APOBEC3C, APOBEC3H, APOBEC3D, APOBEC3F, APOBEC3B, APOBEC3A, APOBEC2 and APOBEC1 (Q9HC16, Q9NRW3, Q6NTF7, Q96AK3, Q8IUX4, Q9UH17, P31941, Q9Y235, and P41238 UniProtKB Entry codes respectively); murine Apobec3, Apobec2, and Apobec1 (Q99J72, Q9Y235, P51908 UniProtKB Entry codes respectively); rat Apobec1 (P38483, UniProtKB Entry code); and derivatives or analogues thereof.

Neither 5caC and 5hmC with a protecting group can be deaminated by APOBEC, and so after the indexing PCR, they will remain as C. Unmodified cytosines (C), since they are not targeted by TET nor BGT, can be then deaminated by APOBEC and become uracils (U).

The cleanup of the ssNA in steps e1) and e2) is essential because if the oligonucleotides of steps f1) and f2) are added while the enzymes responsible of the enzymatic deamination mentioned above are still active they will modify the nucleotide sequence of said primers making the annealing impossible. Another advantage of the present invention is that fully hydroxymethylated adapters are not needed, as they are expensive and difficult to synthesize.

The method of the invention may additionally comprise inactivating the one or more enzymes mentioned above (APOBEC, TET and/or BGT) by providing a protease, by a change in temperature, and/or by a change in pH, for instance, the protease is selected from the group consisting of trypsin, proteinase K, endoproteinase AspN, endoproteinase GluC, furin, enterokinase, factor Xa, and subtilisin.

In a particular embodiment, the inactivation of one or more of the enzymes selected from the group consisting of: APOBEC, TET and BGT can be done by providing a chemical compound suitable to block said enzymes function, preferably EDTA.

In a particular embodiment the inactivation of the enzymes is performed after steps b) and/or c).

In a particular embodiment, the method of the invention comprises an additional step between steps e1)/e2) and f1)/f2) respectively wherein the ssNA is combined with SSB.

In another particular embodiment, the ssNA is combined with SSB after steps d1)/d2). SSB avoids rehybridization of the ssNA when the temperature drops to 37°C after the denaturation step at high temperature.

The ssNA of steps b) and c) have to be separately combined with single-stranded nucleic acid binding protein (SSB) to produce SSB-bound ssNA. SSB binds in a cooperative manner to ssNA and does not bind well to double-stranded nucleic acid (dsNA). Upon binding ssDNA, SSB destabilizes helical duplexes. The advantage of SSBs is that it keeps the DNA available as a single strand so it can hybridize with the oligonucleotides of steps f1) and f2) and avoid ssNA molecules to hybridize among themselves.

SSB is particularly useful when the nucleic acid comprises highly repetitive sequences, as there is higher probability that ssNA with said sequences will renature or form complex secondary structures, thus reducing the yield of the following steps.

In a particular embodiment, SSB is particularly useful when there has been a previous enrichment step in the nucleic acid sample for a target region of the genome as these target regions usually have similar sequences.

In a particular embodiment SSB includes prokaryotic SSB (e.g., bacterial or archaeal SSB) and eukaryotic SSB. Non-limiting examples of SSBs that may be employed include *E. coli* SSB (P0AGE0), *E. coli* RecA, Extreme Thermostable Single-Stranded DNA Binding Protein (ET SSB), *Thermus thermophilus* (Tth) RecA, T4 Gene 32 Protein, replication protein A (RPA - a eukaryotic SSB), and the like. ET SSB, Tth RecA, *E. coli* RecA, T4 Gene 32 Protein, as well buffers and detailed protocols for preparing SSS-bound ssNA using such SSBs are available from, e.g., New England Biolabs, Inc. (Ipswich, MA). Given equal molarity inputs, a greater input of SSB is beneficial for ssNAs with higher average fragment lengths.

In steps f1) and/or f2) the ssNA can be alone or SSB-bound ssNA.

In step f1) and/or f2), the ssNA of step d1) and/or d2) or the SSB-bound ssNA, is combined with a
a first adapter-splinted oligonucleotide complex, and
a second adapter-splinted oligonucleotide complex.

These oligonucleotide complexes comprise:
a first adapter oligonucleotide, and a first splint oligonucleotide comprising an ssNA hybridization region and a first adapter oligonucleotide hybridization region, and
a second adapter oligonucleotide, and a second splint oligonucleotide comprising an ssNA hybridization region and a second adapter oligonucleotide hybridization region,
having each oligonucleotide one or more blocking modifications at the end under conditions to form complexes comprising
the first splint oligonucleotide hybridized to a terminal region of the ssNA via the ssNA hybridization region, and the first splint oligonucleotide hybridized to the first adapter oligonucleotide via the first adapter oligonucleotide hybridization region, such that an end of the first adapter oligonucleotide is adjacent to an end of the terminal region of the ssNA, and
the second splint oligonucleotide hybridized via the ssNA hybridization region to the terminal region of the ssNA opposite the terminal region hybridized to the first splint oligonucleotide, and the second splint oligonucleotide hybridized to the second adapter oligonucleotide via the second adapter oligonucleotide hybridization region, such that an end of the second adapter oligonucleotide is adjacent to the end of the ssNA opposite the end adjacent to the first adapter,

A first adapter oligonucleotide, and a first splint oligonucleotide including an ssNA hybridization region and a first adapter oligonucleotide hybridization region are hybridized to form a first adapter-splinted oligonucleotide complex before step f1) or f2). The first adapter-splinted oligonucleotide complex hybridizes to a terminal region of the ssNA via the ssNA hybridization region, and the first splint oligonucleotide hybridized to the first adapter oligonucleotide via the first adapter oligonucleotide hybridization region, such that an end of the first adapter oligonucleotide is adjacent to an end of the first terminal region of the ssNA.

A second adapter oligonucleotide, and a second splint oligonucleotide including an ssNA hybridization region and a second adapter oligonucleotide hybridization region, are hybridized to form a second adapter-splinted oligonucleotide complex before step f1) or f2). The second adapter-splinted oligonucleotide complex hybridizes via the ssNA hybridization region to the terminal region of the ssNA opposite the terminal region hybridized to the first splint oligonucleotide, and the second splint oligonucleotide hybridized to the second adapter oligonucleotide via the second adapter oligonucleotide hybridization region, such that an end of the second adapter oligonucleotide is adjacent to the end of the ssNA opposite the end adjacent to the first adapter oligonucleotide.

The formation of the complexes need to be done before steps f1) and f2) as for by enzymatic ligation with T4 DNA ligase to work in the hybridization prior to steps f1) and f2), the two nucleic acid fragments being ligated have to be double stranded. If the ssNA, the first/second adapter oligonucleotide and the first/second splinted oligonucleotide were combined together without previously forming the oligonucleotide complexes the ligation yield will be greatly reduced.

The conditions to form the adapter-splinted oligonucleotide complexes comprise a first adapter oligonucleotide in a final concentration between 8 to 15 µM was combined with the first splint oligonucleotide to a final concentration of 8 to 15 µM with 1 X final concentration of T4 RNA Ligase Buffer (1X T4 RNA Ligase Buffer Components: 50 mM Tris-HCI, 10 mM MgCl₂, 1 mM DTT and pH 7.5 at 25°C). A separate mixture was prepared using the second adapter oligonucleotide and the second splint oligonucleotide. The oligos were hybridized by heating to 95°C for 10 seconds and then and then ramped down to 10°C at a rate of 0.1°C/s.

The splint oligonucleotides are designed such that when the ssNA hybridization regions of the splint oligonucleotides are hybridized to their respective terminal regions of the ssNA, an end of the adapter oligonucleotide is adjacent to an end of the ssNA. These adjacent ends may be covalently linked (e.g., by enzymatic ligation, for instance, by T4 DNA ligase) to produce adapted ssNA which may then be used in a downstream application of interest (e.g., PCR amplification, next-generation sequencing, and/or the like) facilitated by one or more sequences in the adapter portion of the adapted ssNA.

An optional clean-up step may be performed to separate the adapted ssNA from one or more reagents or components of the formed complexes, e.g., enzyme used for the covalent linkage, splint oligonucleotides, SSB, and/or the like. Suitable approaches for such a clean-up step include, but are not limited to, solid phase reversible immobilization (SPRI - e.g., using magnetic beads) and nucleic acid column purification.

An "oligonucleotide" is a single-stranded or double-stranded multimer of nucleotides from 5 to 500 nucleotides, e.g., 5 to 100 nucleotides. Oligonucleotides may be synthetic or may be made enzymatically, and, in some embodiments, are 5 to 50 nucleotides in length. Oligonucleotides may contain ribonucleotide monomers (i.e., may be oligoribonucleotides or "RNA oligonucleotides"), deoxyribonucleotide monomers (i.e., may be oligodeoxyribonucleotides or "DNA oligonucleotides"), or a combination thereof. Oligonucleotides may be 10 to 20, 20 to 30, 30 to 40, 40 to 50, 50 to 60, 60 to 70, 70 to 80, 80 to 100, 100 to 150 or 150 to 200, or up to 500 nucleotides in length, for example.

An "adapter oligonucleotide" is an oligonucleotide that includes an adapter or portion thereof. By "adapter" is meant a nucleotide sequence useful for one or more downstream applications (e.g., PCR amplification of the adapted ssNA or derivative thereof, sequencing of the adapted ssNA or derivative thereof, and/or the like). In certain aspects, the adapter or portion thereof present in the adapter oligonucleotide is a sequencing adapter. By "sequencing adapter" is meant one or more nucleic acid domains that include at least a portion of a nucleotide sequence (or complement thereof) utilized by a sequencing platform of interest, such as a sequencing platform provided by Illumina^{®} (e.g., the HiSeq^{™}, MiSeq^{™} and/or Genome Analyzer^{™} sequencing systems); Oxford Nanopore^{™} Technologies (e.g., the MinION^{™} sequencing system), Ion Torrent^{™} (e.g., the Ion PGM^{™} and/or Ion Proton^{™} sequencing systems); Pacific Biosciences (e.g., a Sequel or PacBio RS II sequencing system); Life Technologies^{™} (e.g., a SOLiD^{™} sequencing system); Roche (e.g., the 454 GS FLX+ and/or GS Junior sequencing systems); or any other sequencing platform of interest. Preferably Illumina^{®} platform, as this is one of the cheapest technology for sequencing and the nucleic acid sample is fragmented in step a) of the methods of the invention so the fragments can be completely sequenced by this technology, as it has a limited length capacity.

In certain aspects, the sequencing adapter is, or includes, a nucleic acid domain selected from: a domain (e.g., a "capture site" or "capture sequence") that specifically binds to a surface-attached sequencing platform oligonucleotide (e.g., the P5 or P7 oligonucleotides attached to the surface of a flow cell in an Illumina^{®} sequencing system); a sequencing primer binding domain (e.g., a domain to which the Read 1 or Read 2 primers of the Illumina^{®} platform may bind); a unique identifier (e.g., a barcode or other domain that uniquely identifies the 3' region of the oligonucleotide probe, the probe complement oligonucleotide, or both, and/or uniquely identifies the sample source of the rRNA being sequenced to enable sample multiplexing by marking every molecule from a given sample with a specific barcode or "tag"); a barcode sequencing primer binding domain (a domain to which a primer used for sequencing a barcode binds); a molecular identification domain (e.g., a molecular index tag, such as a randomized tag of 4, 6, or other number of nucleotides) for uniquely marking molecules of interest, e.g., to determine expression levels based on the number of instances a unique tag is sequenced; a complement of any such domains; or any combination thereof. In certain aspects, a barcode domain (e.g., sample index tag) and a molecular identification domain (e.g., a molecular index tag) may be included in the same nucleic acid.

When the adapter oligonucleotide includes one or a portion of a sequencing adapter, one or more additional sequencing adapters and/or a remaining portion of the sequencing adapter may be added using a variety of approaches. For example, additional and/or remaining portions of sequencing adapters may be added by ligation, reverse transcription, PCR amplification, and/or the like. In the case of PCR, an amplification primer pair may be employed that includes a first amplification primer that includes a 3' hybridization region (e.g., for hybridizing to an adapter region of the adapter oligonucleotide) and a 5' region including an additional and/or remaining portion of a sequencing adapter, and a second amplification primer that includes a 3' hybridization region (e.g., for hybridizing to an adapter region of a second adapter oligonucleotide added to the opposite end of an ssNA molecule) and optionally a 5' region including an additional and/or remaining portion of a sequencing adapter.

In a particular embodiment, the first adapter oligonucleotide sequence is SEQ ID NO:1 polynucleotide sequence (5'-3'):
ACACTCTTTCCCTACACGACGCTCTTCCGATCT, or a variant having at least 80 % identity, more preferably 85% identity, even more preferably at least 90 % identity, and even more preferably 93 % or 97 % or even up to 97 % identity with respect to the SEQ ID NO:1 polynucleotide sequence.

In another particular embodiment, the second adapter oligonucleotide sequence is SEQ ID NO:2 polynucleotide sequence (5'-3'):
AGATCGGAAGAGCACACGTCTGAACTCCAGTCAC, or a variant having at least 80 % identity, more preferably 85% identity, even more preferably at least 90 % identity, and even more preferably 93 % or 97 % or even up to 97 % identity with respect to the SEQ ID NO:2 polynucleotide sequence.

A "splint oligonucleotide" of the present disclosure is an oligonucleotide that includes an ssNA hybridization region and an adapter oligonucleotide hybridization region. The ssNA hybridization region is a region (nucleotide sequence) that hybridizes to a terminal region of the ssNA. The adapter oligonucleotide hybridization region is a region (nucleotide sequence) that hybridizes to all or a portion of the adapter oligonucleotide. The splint oligonucleotide is designed for simultaneous hybridization to the ssNA and the adapter oligonucleotide such that, upon complex formation, an end of the adapter oligonucleotide is adjacent to an end of the terminal region of the ssNA.

The ssNA hybridization region of the splint oligonucleotide may have any suitable length and sequence. In some embodiments, the length of the ssNA hybridization region is 10 nucleotides or less. In certain aspects, the ssNA hybridization region is from 4 to 20 nucleotides in length, e.g., from 5 to 15, 5 to 10, 5 to 9, 5 to 8, or 5 to 7 (e.g., 6 or 7) nucleotides in length. In some embodiments, the ssNA hybridization region includes (e.g., consists of) a random nucleotide sequence, such that when a plurality of heterogeneous splint oligonucleotides having various random ssNA hybridization regions are employed, the collection is capable of acting as splint oligonucleotides for a heterogeneous population of ssNAs irrespective of the sequences of the terminal regions of the ssNAs.

In a particular embodiment, the first splint oligonucleotide sequence is SEQ ID NO:3 polynucleotide sequence (5'-3'):
NNNNNNNAGATCGGAAGAGCGTCGTGTAGGGAAAGAGTGT,

Wherein "N" is a nucleotide selected from the group consisting of A, C, T, G. This part is the ssNA hybridization region. A random combination of 7 "N" will cover all options to hybridize with ssNA nucleotide sequences covering the whole genome, this is, a random sequence.

The ssNA hybridization region may comprise a random sequence and/or a universal base.

And "AGATCGGAAGAGCGTCGTGTAGGGAAAGAGTGT" is the adapter oligonucleotide hybridization region.

In another particular embodiment, the adapter oligonucleotide hybridization region of the first splint oligonucleotide sequence is a variant having at least 80 % identity, more preferably 85% identity, even more preferably at least 90 % identity, and even more preferably 93 % or 97 % or even up to 97 % identity with respect to the SEQ ID NO:3 polynucleotide sequence, concretely the adapter oligonucleotide hybridization region.

In another particular embodiment, the second splint oligonucleotide sequence is SEQ ID NO:4 polynucleotide sequence (5'-3'):
GTGACTGGAGTTCAGACGTGTGCTCTTCCGATCTNNNNNNN,

Wherein "N" is a nucleotide selected from the group consisting of A, C, T, G. This part is the ssNA hybridization region. A random combination of 7 "N" will cover all options to hybridize with ssNA nucleotide sequences covering the whole genome, this is a random sequence.

The ssNA hybridization region may comprise a random sequence and/or a universal base.

And "GTGACTGGAGTTCAGACGTGTGCTCTTCCGATCT" is the adapter oligonucleotide hybridization region.

In another particular embodiment, the adapter oligonucleotide hybridization region of the first splint oligonucleotide sequence is a variant having at least 80 % identity, more preferably 85% identity, even more preferably at least 90 % identity, and even more preferably 93 % or 97 % or even up to 97 % identity with respect to the SEQ ID NO:4 polynucleotide sequence, concretely the adapter oligonucleotide hybridization region.

It is important that the variants with respect:
First adaptor oligonucleotide SEQ ID NO:1 and the first splint oligonucleotide SEQ ID NO:3, and
second adaptor oligonucleotide SEQ ID NO:2, and the second splint oligonucleotide SEQ ID NO:4,
are designed so that the hybridization region between those oligonucleotides is functional and they can hybridize.

In some embodiments, the ssNA hybridization region includes a known sequence designed to hybridize to a ssNA terminal region of known sequence. In certain aspects, two or more heterogeneous splint oligonucleotides having different ssNA hybridization regions of known sequence designed to hybridize to respective ssNA terminal regions of known sequence are employed. Embodiments in which the ssNA hybridization regions have a known sequence find use, e.g., when it is desirable to produce a nucleic acid library from only a subset of ssNAs having terminal regions of known sequence. Accordingly, in certain aspects, the methods include forming the complexes by combining the ssNA, an adapter oligonucleotide, and one or more heterogeneous splint oligonucleotides having one or more different ssNA hybridization regions of known sequence capable of acting as splint oligonucleotides for one or more ssNAs having one or more terminal regions of known sequence.

The manner in which the ssNA, the adapter oligonucleotide, and the splint oligonucleotide are combined may vary. The combining is carried out under hybridization conditions such that complexes including the splint oligonucleotide hybridized to a terminal region of the ssNA via the ssNA hybridization region, and the splint oligonucleotide hybridized to the adapter oligonucleotide via the adapter oligonucleotide hybridization region. Whether specific hybridization occurs is determined by such factors as the degree of complementarity between the relevant (that is, hybridizing) regions of the splint oligonucleotide, the terminal region of the ssNA, and the adapter oligonucleotide, as well as the length thereof, salt concentration, and the temperature at which the hybridization occurs, which may be informed by the melting temperatures (T*_{M}*) of the relevant regions. The melting temperature refers to the temperature at which half of the relevant regions remain hybridized and half of the relevant regions dissociate into single strands. The *Tm* of a duplex may be experimentally determined or predicted using the following formula *Tm* = 81.5 + 16.6(log¹⁰[Na⁺]) + 0.41 (fraction G+C) - (60/N), where N is the chain length and [Na⁺] is less than 1M. See Sambrook and Russell (2001; Molecular Cloning: A Laboratory Manual, 3rd ed., Cold Spring Harbor Press, Cold Spring Harbor N.Y., Ch. 10). Other more advanced models that depend on various parameters may also be used to predict *Tm* of relevant regions depending on various hybridization conditions. Approaches for achieving specific nucleic acid hybridization may be found in, e.g., Tijssen, Laboratory Techniques in Biochemistry and Molecular Biology-Hybridization with Nucleic Acid Probes, part I, chapter 2, Overview of principles of hybridization and the strategy of nucleic acid probe assays," Elsevier (1993).

The terms "complementary" or "complementarity" as used herein refer to a nucleotide sequence that base-pairs by non-covalent bonds to a region of a target nucleic acid. These terms may also encompass duplexes that are fully complementary such that every nucleotide in one strand is complementary to every nucleotide in the other strand in corresponding positions. In certain cases, a nucleotide sequence may be partially complementary to a target, in which not all nucleotides are complementary to every nucleotide in the target nucleic acid in all the corresponding positions. For example, the ssNA hybridization region may be perfectly (i.e., 100%) complementary to the terminal region of the ssNA, or the ssNA hybridization region may share some degree of complementarity which is less than perfect (e.g., 70%, 75%, 85%, 90%, 95%, 99%). The percent identity of two nucleotide sequences can be determined by aligning the sequences for optimal comparison purposes (e.g., gaps can be introduced in the sequence of a first sequence for optimal alignment). A skilled in the art would know how to determine the degree of complementarity by using routine analyses such as BLAST software.

The complexes are formed such that an end of the adapter oligonucleotide is adjacent to an end of the terminal region of the ssNA. By "adjacent to" is meant the terminal nucleotide at the end of the adapter oligonucleotide and the terminal nucleotide end of the terminal region of the ssNA are sufficiently proximal to each other that the terminal nucleotides may be covalently linked, e.g., by chemical ligation, enzymatic ligation, or the like. In some embodiments, the ends are adjacent to each other by virtue of the terminal nucleotide at the end of the adapter oligonucleotide and the terminal nucleotide end of the terminal region of the ssNA being hybridized to adjacent nucleotides of the splint oligonucleotide. The splint oligonucleotide may be designed to ensure that an end of the adapter oligonucleotide is adjacent to an end of the terminal region of the ssNA. Non-limiting examples of such splint oligonucleotides are provided in the Experimental section herein.

Any of the methods described herein may further include covalently linking the adjacent ends of an adapter oligonucleotide and ssNA. The covalent linking may include ligating the adjacent ends. Ligating the adjacent ends may be carried out using any suitable approach. In certain aspects, the ligating is by chemical ligation. In other aspects, the ligating is by enzymatic ligation. Suitable reagents (e.g., ligases and corresponding buffers, etc.) and kits for performing enzymatic ligation reactions are known and available, e.g., the Instant Sticky-end Ligase Master Mix available from New England Biolabs (Ipswich, MA). Ligases that may be employed include, e.g., T4 DNA ligase (e.g., at low or high concentration), T4 DNA ligase, T7 DNA Ligase, E. coli DNA Ligase, Electro Ligase^{®}, or the like. Conditions suitable for performing the ligation reaction will vary depending upon the type of ligase used. Information regarding such conditions is readily available. When necessary, a phosphate group may be added at the 5' end of the adapter oligonucleotide or ssNA using, e.g., a suitable kinase, such as T4 polynucleotide kinase (PNK). Such kinases and guidance for using such kinases to phosphorylate 5' ends are available, e.g., from New England BioLabs, Inc. (Ipswich, MA).

In some embodiments, the splint oligonucleotide, the adapter oligonucleotide, or both, includes a blocking modification. For example, one or both ends of the splint oligonucleotide may include a blocking modification and/or the end of the adapter oligonucleotide not adjacent to the ssNA may include a blocking modification. By "blocking modification" is meant the end is not competent for being linked to the end of any other oligonucleotide components using an approach employed to covalently link the adjacent ends of the adapter oligonucleotide and ssNA. In certain aspects, the blocking modification is a ligation-blocking modification. Examples of blocking modifications which may be included at one or both ends of the splint oligonucleotide and/or the end of the adapter oligonucleotide not adjacent to the ssNA, include the absence of a 3' OH at the end of the adapter oligonucleotide not adjacent to the ssNA, and an inaccessible 3' OH at the end of the adapter oligonucleotide not adjacent to the ssNA. Non-limiting examples of blocking modifications in which an end has an inaccessible 3' OH include: an amino modifier, a spacer, a dideoxy base, an inverted dideoxy base, a 3' phosphate, or the like.

In a particular embodiment the first adapter oligonucleotide (SEQ ID NO:1) has a 5'Amino Modifier C12 in the 5' end.

In another particular embodiment the second adapter oligonucleotide (SEQ ID NO:2) has a 5'Phosphate modification in the 5' end and/or a 3'Dideoxy Cytosine in the 3' end.

In another particular embodiment the first splint oligonucleotide (SEQ ID NO:3) has a 5'Amino Modifier C6 in the 5' end and/or a 3' Amino Modifier in the 3' end.

In another particular embodiment the second splint oligonucleotide (SEQ ID NO:4) has a 5'Amino Modifier C6 in the 5' end and/or a 3' Amino Modifier in the 3' end.

Covalently linking the adjacent ends of an adapter oligonucleotide and ssNA produces adapted ssNA, where "adapted" means the ssNA now includes one or more adapter sequences or subregions thereof. The adapted ssNA may be purified before being used as input in a downstream application of interest. For example, the complexes may be denatured (e.g., heat-denatured) to separate the adapted ssNA from the splint oligonucleotides, the adapted ssNA may be purified from the SSB and/or any other components present during the contacting and/or combining steps (e.g., by solid phase reversible immobilization (SPRI), column purification, and/or the like), or combinations thereof.

In a particular embodiment the adjacent ends of the first adapter oligonucleotide and ssNA are covalently linked and the adjacent ends of the second adapter oligonucleotide and ssNA are covalently linked, the covalent linking of the adjacent ends is performed by enzymatic ligation.

In another particular embodiment, the conditions of step f1) and/or f2) are a temperature range between 25°C and 45 °C, preferably between 30°C and 38°C during a period of time between 30 minutes and 180 hours, preferably 40 min and 90 minutes.

In another particular embodiment steps f1) and f2) comprises combining:
a complex comprising the first splint oligonucleotide hybridized to the first adapter oligonucleotide via the first adapter oligonucleotide hybridization region,
a complex comprising the second splint oligonucleotide hybridized to the second adapter oligonucleotide via the second adapter oligonucleotide hybridization region, and the ssNA.

In another particular embodiment steps f1) and f2) comprises combining:
a complex comprising the first splint oligonucleotide hybridized to the ssNA via the ssNA hybridization region,
a complex comprising the second splint oligonucleotide hybridized to the ssNA via the ssNA hybridization region,
the first adapter oligonucleotide, and
the second adapter oligonucleotide.

In another particular embodiment the adapter oligonucleotide comprises an adapter for PCR amplification or a complement thereof; and/or a partial or complete sequencing adapter or a complement thereof.

In a particular embodiment, steps g1) and g2) are performed as soon as possible after steps f1) and f2) are finished in order to maximize the production yield of dsDNA nucleic acid libraries and without a temperature change.

In a particular embodiment "as soon as possible" is less than 6hr, preferably, less the 4hr, more preferably less than 2hr, even more preferably less than 1hr, even more preferably after finishing steps f1) and f2).

The products of steps f1) and f2) should not be subjected to a temperature change, this is frozen or storage at low temperatures (less than 5°C, preferably less than 10°C, even more preferably less then 15°C) before being amplified by indexing PCR in steps g1) and g2) because it will decrease the amount of dsDNA nucleic acid libraries obtained.

The method of the invention further includes, in steps g1) and g2), after formation of the complexes, rehybridizing the ssNA (which now includes one or more adapters (e.g., sequencing adapters) at one or both ends) to produce dsDNA library which may be sequenced. In some embodiments, the rehybridizing is carried out under sufficiently stringent hybridization conditions to produce dsDNAs that resemble the original dsDNAs from which the ssDNA was derived. The sufficiently stringent hybridization conditions may include a selected hybridization temperature, a selected salt concentration, and/or any other convenient hybridization parameters selected to produce dsDNAs that resemble the original dsDNAs from which the ssNA was derived.

In a particular embodiment the ds DNA library is made by an indexing PCR wherein the sequencing adapters are P5 and P7 index primers
SEQ ID NO:5 P5 Index primer: 5'- 
AATGATACGGCGACCACCGAGATCTACACNNNNNNNACACTCTTTCCCTACACGA
CGCTCTT-3'
SEQ ID NO:6 P7 Index primer: 5'-CAAGCAGAAGACGGCATACGAGATNNNNNNNGTGACTGGAGTTCAGACGTGT-3'

Wherein "N" is a nucleotide selected from the group consisting of A, C, T, G. This region is termed "index". A given combination of 6 to 8 "N" of SEQ ID NO:5 and SEQ ID NO:6 will allow to determine in the subsequent step of sequencing to which sample does a particular read belong to, when all samples are sequencing together. A skilled in the field would know the possibilities of given combinations of "N" as they are known in the art.

The index sequences of the sequencing adapters are different for each nucleic acid sample. They have the common Illumina sequence but a different index for each library, so they are added to each sample separately.

In another particular embodiment, the region comprised between nucleotides 1 to 29 and/or the region comprised between nucleotides 37 to 62 of the P5 indexing primer sequence is a variant having at least 80 % identity, more preferably 85% identity, even more preferably at least 90 % identity, and even more preferably 93 % or 97 % or even up to 97 % identity with respect to the SEQ ID NO:5 polynucleotide sequence.

In another particular embodiment, the region comprised between nucleotides 1 to 24 and/or the region comprised between nucleotides 32 to 52 of the P7 indexing primer sequence is a variant having at least 80 % identity, more preferably 85% identity, even more preferably at least 90 % identity, and even more preferably 93 % or 97 % or even up to 97 % identity with respect to the SEQ ID NO:6 polynucleotide sequence.

In a particular embodiment the method of the invention further comprises the step of amplifying(increasing) the copy number of a target nucleic acid sequence within the dsDNA nucleic acid libraries obtained in steps g1) and/or g2) by methods known in the art. The copy number can be increased by, for example, PCR, cloning, and primer extension. The copy number of individual target nucleic acids can be amplified by PCR using primers specific for a particular target nucleic acid sequence.

In another particular embodiment, the quality and quantity of the dsDNA nucleic acid libraries is checked before continuing with step h). The quality and quantity can be verified by any method know in the art used to measure nucleic acids.

The steps h1) and h2) of the method of the invention comprises the detection of the sequence of the modified target nucleic acid. The modified DNA of the dsDNA library contains (T) at original (T) positions and at original (C) positions methylated or hydroxymethylated that were not protected in the earlier steps b) and/or c), and were thus subsequently deaminated in steps e1) and e2) and converted into (U). The (U) can be detected as a C to T transition. U acts as a T in DNA replication and sequencing methods. Thus, the cytosine modifications can be detected by any direct or indirect method that identifies a C to T transition known in the art. Such methods include sequencing methods such as Sanger sequencing, microarray, and next generation sequencing methods, including, but not limited to single-molecule real-time (SMRT) sequencing, Ion semiconductor sequencing (Ion Torrent sequencing), sequencing by synthesis (Illumina), combinatorial probe anchor synthesis (cPAS-BGI/MGI), sequencing by ligation (SOLiD sequencing), and nanopore sequencing. In particular, the method described herein are useful for detecting cytosine modifications using a long-read sequencing technology including PacBio SingleMolecule Real-Time (SMRT) sequencing and Oxford Nanopore sequencing.

In some embodiments, the one or more adapter sequences or subregions thereof is one or more sequencing adapters or subregions thereof, and the methods further include sequencing at least a portion of the adapted ssNA, or any derivative thereof (e.g., amplicons produced by PCR amplification using the adapted ssNA as template). The sequencing may be carried out on any suitable sequencing platform, including a high-throughput sequencing (HTS) (or "next-generation sequencing (NGS)") platform, or the like. HTS/NGS sequencing platforms of interest include, but are not limited to, a sequencing platform provided by Illumina^{®} (e.g., the HiSeq^{™}, MiSeq^{™} and/or Genome Analyzer^{™} sequencing systems); Oxford Nanopore^{™} Technologies (e.g., a MinION^{™}, GridION×5^{™}, PromethION^{™}, or SmidgION^{™} nanopore-based sequencing system), Ion Torrent^{™} (e.g., the Ion PGM^{™} and/or Ion Proton^{™} sequencing systems); Pacific Biosciences (e.g., a Sequel or PacBio RS II sequencing system); Life Technologies^{™} (e.g., a SOLiD sequencing system); Roche (e.g., the 454 GS FLX+ and/or GS Junior sequencing systems); or any other sequencing platform of interest. Detailed protocols for direct sequencing (e.g., by nanopore-based sequencing) or preparing compatible nucleic acid molecules for sequencing on a particular platform (e.g., by amplification, e.g., solid-phase amplification, or the like), sequencing the compatible molecules, and analyzing the sequencing data are available from the manufacturer of the sequencing platform of interest.

Thus, in the method to detect 5-hydroxymethylcytosine (5hmC) in a nucleic acid sample (EOH-seq), after step h1), the comparison of the sequencing results of dsDNA library compared to the original nucleic acid sequence of step a) provides the location of 5hmC in the initial nucleic acid sample of step a):
- unmodified (C) will be observed as (T) in the dsDNA library compared to the original nucleic acid sequence of step a) that will have a (C) in the same position
- methylated cytosines (5mC) will be observed as (T) in the dsDNA library, compared to the original nucleic acid sequence of step a) that will have a (C) in the same position
- hydroxymethylated cytosines (5hmC) will be observed as (C) in the dsDNA library, compared to the original nucleic acid sequence of step a) that will have a (C) in the same position.

In the method to detect 5-methylcytosine (5mC) and 5-hydroxymethylcytosine (5hmC) in a nucleic acid sample (sEM-seq), after step h2), the comparison of the sequencing results of dsDNA library compared to the original nucleic acid sequence of step a) provides the location of 5mC or 5hmC in the initial nucleic acid sample of step a):
- unmodified (C) will be observed as (T) in the dsDNA library compared to the original nucleic acid sequence of step a) that will have a (C) in the same position
- methylated cytosines (5mC) will be observed as (C) in the dsDNA library, compared to the original nucleic acid sequence of step a) that will have a (C) in the same position
- hydroxymethylated cytosines (5hmC) will be observed as (C) in the dsDNA library, compared to the original nucleic acid sequence of step a) that will have a (C) in the same position

In the method to detect and to discriminate 5-methylcytosine (5mC) from 5-hydroxymethylcytosine (5hmC) in a nucleic acid sample (M-seq), in step h), the comparison of the sequencing results of both nucleic acid samples in ((1) and (2)) and the original nucleic acid sequence provides the location of 5hmC and 5mC:
- unmodified (C) will be observed as (T) in the dsDNA library processed as (1) and in the dsDNA library processed as (2), compared to the original nucleic acid sample sequence that will have a (C) in the same position
- methylated cytosines (5mC) will be observed as (T) in the dsDNA library processed as (1), compared to the original nucleic acid sample sequence, and in the dsDNA library processed as (2) that will have a (C) in the same position.
- hydroxymethylated cytosines (5hmC) will be observed as (C) in the dsDNA library processed as (1), in the dsDNA library processed as (2) and in the original nucleic acid sample sequence

Calculating the percentages of C or T at each particular position in step h1) and h2) provides a quantitative level of 5mC or 5hmC at each location in the nucleic acid of the whole genome.

In a particular embodiment the sequencing of dsDNA libraries of (1) and (2) can be performed together in the same tube and/or platform because as they were separately amplified in steps f1) and f2) with indexed primers with different tags, a skilled in the art, by looking at the sequencing results can differentiate between results from (1) and (2).

Another object of the present invention relates to a Kit for detecting 5-hydroxymethylcytosine (5hmC) in a nucleic acid sample (EOH-seq) and/or for detecting 5-methylcytosine (5mC) and 5-hydroxymethylcytosine (5hmC) in a nucleic acid sample (sEM-seq) and/or for detecting and discriminating 5-methylcytosine (5mC) and 5-hydroxymethylcytosine (5hmC) in a nucleic acid sample (M-seq). Such kits comprise reagents for detecting 5-hydroxymethylcytosine (5hmC) in a nucleic acid sample and/or for detecting 5-methylcytosine (5mC) and 5-hydroxymethylcytosine (5hmC) in a nucleic acid sample and/or for identification of 5mC and/or 5hmC by the methods described herein.

In a particular embodiment, the kit comprises a TET enzyme, a BGT enzyme (for instance, T4-α-glucosyltransferase and/or T4-β- glucosyltransferase), and APOBEC enzyme as described above and instructions for performing the method.

the kit further comprises a 5hmC blocking group and a glucosyltransferase enzyme. In further embodiments, the 5hmC blocking group is uridine di phosphate (UDP)-sugar where the sugar is glucose or a glucose derivative, and the glucosyltransferase enzyme is T4 bacteriophage β- or α-glucosyltransferase, and derivatives and analogs thereof. In embodiments, the kit comprises reagents for isolating DNA or RNA. In embodiments the kit comprises reagents for isolating low-input DNA from a sample, for example cfDNA from blood, plasma, or serum.

In another particular embodiment, the kit further includes single-stranded nucleic acid binding protein (SSB), a first adapter oligonucleotide, a first splint oligonucleotide comprising an SSB-bound ssNA hybridization region and a first adapter oligonucleotide hybridization region. It further includes a second adapter oligonucleotide, and a second splint oligonucleotide including an SSB-bound ssNA hybridization region and a second adapter oligonucleotide hybridization region, where the instructions are for using the SSB, first adapter oligonucleotide, first splint oligonucleotide, second adapter oligonucleotide, and second splint oligonucleotide to produce a nucleic acid library.

The kits of the present disclosure may further include a reagent for covalently linking an adapter oligonucleotide end to an end of SSB-bound ssNA. In some embodiments, the reagent is a chemical ligation reagent or an enzymatic ligation reagent, e.g., a ligase.

In another particular embodiment, the oligonucleotides include a blocking modification. For example, one or both ends of the splint oligonucleotide may include a blocking modification and/or the end of the adapter oligonucleotide not adjacent to the SSB-bound ssNA may include a blocking modification. In certain aspects, the blocking modification is a ligation-blocking modification. Examples of blocking modifications which may be included at one or both ends of the splint oligonucleotide and/or the end of the adapter oligonucleotide not adjacent to the SSB-bound ssNA, include the absence of a 3' OH at the end of the adapter oligonucleotide not adjacent to the SSB-bound ssNA, and an inaccessible 3' OH at the end of the adapter oligonucleotide not adjacent to the SSB-bound ssNA. Non-limiting examples of blocking modifications in which an end has an inaccessible 3' OH include: an amino modifier, a spacer, a dideoxy base, an inverted dideoxy base, a 3' phosphate, or the like.

Components of the subject kits may be present in separate containers, or multiple components may be present in a single container. A suitable container includes a single tube (e.g., vial), one or more wells of a plate (e.g., a 96-well plate, a 384-well plate, etc.), or the like.

The instructions for using the SSB, one or more adapter oligonucleotides, and one or more splint oligonucleotides to produce a nucleic acid library may be recorded on a suitable recording medium. For example, the instructions may be printed on a substrate, such as paper or plastic, etc. As such, the instructions may be present in the kits as a package insert, in the labeling of the container of the kit or components thereof (i.e., associated with the packaging or sub-packaging), etc. In other embodiments, the instructions are present as an electronic storage data file present on a suitable computer-readable storage medium, e.g., portable flash drive, DVD, CD-ROM, diskette, etc. In yet other embodiments, the actual instructions are not present in the kit, but means for obtaining the instructions from a remote source, e.g. via the internet, are provided. An example of this embodiment is a kit that includes a web address where the instructions can be viewed and/or from which the instructions can be downloaded. As with the instructions, the means for obtaining the instructions is recorded on a suitable substrate.

In addition, the present invention also provides the following clauses:
1. A method to detect 5-hydroxymethylcytosine (5hmC) in a nucleic acid sample, comprising the following steps:
   **a)** providing a nucleic acid sample, said nucleic acid sample being
      a genomic DNA sample or
      a genomic DNA sample that has gone through a previous enrichment step for a target region of the genome,
      and fragmenting said nucleic acid sample into fragments of a sequence length between 100 base pairs and 10000 base pairs,
   **b)** adding a protecting group to the 5-hydroxymethylcytosine (5hmC) in the fragmented nucleic acid sample of step a),
   **d1)** denaturing the product of step b) to obtain a modified single stranded nucleic acid sample (ssNA),
   **e1)** performing enzymatic deamination of the ssNA product obtained in step d1) to convert cytosines (C) into uracils (U), and performing cleanup of said deaminated ssNA
   **f1)** combining the products of step (e) with
      a first adapter-splinted oligonucleotide complex, and
      a second adapter-splinted oligonucleotide complex
      by enzymatic ligation wherein the first and second adapter-splinted oligonucleotide complex hybridize to the ssNA via a ssNA hybridization region,
   **g1)** amplifying by indexing PCR the products of step f1) by combining said products with at least two different sequencing adapters, to obtain a dsDNA nucleic acid library and
   **h1)** sequencing the nucleic acid sample contained in the dsDNA nucleic acid library of step g1),
   wherein the presence of a cytosine (C) in the same position of the dsDNA nucleic acid library and in the original nucleic acid sequence of step a) provides the location of 5hmC in a nucleic acid sample.
2. The method according to the preceding clause, wherein the percentages of (C) at each position provide a quantitative level of 5hmC at each location of the nucleic acid.
3. A method to detect 5-methylcytosine (5mC) and 5-hydroxymethylcytosine (5hmC) in a nucleic acid sample comprising:
   **a)** providing a nucleic acid sample, said nucleic acid sample being
      a genomic DNA sample or
      a genomic DNA sample that has gone through a previous enrichment step for a target region of the genome,
      and fragmenting said nucleic acid sample into fragments of a sequence length between 100 base pairs and 10000 base pairs,
   **c)** adding a protecting group to the 5-hydroxymethylcytosine (5hmC) and oxidizing the 5-methylcytosine (5mC) of said fragmented second nucleic acid sample
   **d2)** denaturing the product of step c) to obtain a modified single stranded nucleic acid sample (ssNA),
   **e2)** performing enzymatic deamination of the ssNA product obtained in step d2) to convert cytosines (C) into uracyls (U), and performing cleanup of said deaminated ssNA
   **f2)** combining the products of step (e2) with
      a first adapter-splinted oligonucleotide complex, and
      a second adapter-splinted oligonucleotide complex
      by enzymatic ligation wherein the first and second adapter-splinted oligonucleotide complex hybridize to the ssNA via a ssNA hybridization region,
   **g2)** amplifying by indexing PCR the products of step f2) by combining said products with at least two different sequencing adapters, to obtain a dsDNA nucleic acid library and
   **h2)** sequencing the nucleic acid sample contained in the dsDNA nucleic acid library of step g2),
   wherein the presence of a cytosine (C) in the same position of the dsDNA nucleic acid library and in the original nucleic acid sequence of step a) provides the location of 5hmC or 5mC in a nucleic acid sample.
4. The method according to the preceding clause, wherein the percentages of (C) at each position provide a quantitative level of 5hmC or 5mC at each location of the nucleic acid.
5. A method to detect and to discriminate 5-methylcytosine (5mC) from 5-hydroxymethylcytosine (5hmC) in a nucleic acid sample comprising:
   providing a nucleic acid sample, said nucleic acid sample being
      a genomic DNA sample or
      a genomic DNA sample that has gone through a previous enrichment step for a target region of the genome,
   fragmenting said nucleic acid sample into fragments of a sequence length between 100 base pairs and 10000 base pairs,
   dividing it into at least a first nucleic acid sample and a second nucleic acid sample
      **1)** subjecting said fragmented first nucleic acid sample to steps b) to h1) of the method of clause 1,
      **2)** subjecting said fragmented second nucleic acid sample to steps c) to h2) of the method of clause 3,
      **3)** comparing the results of steps (1), (2), wherein the presence of a cytosine (C) in the sequence of the dsDNA nucleic acid library of either (1) and/or (2), and/or in the original nucleic acid sequence of step a) provides the location and discrimination of 5-methylcytosine (5mC) from 5-hydroxymethylcytosine (5hmC).
6. The method according to the preceding clause wherein the products of steps g1) and g2) are combined together for the sequencing.
7. The method according to the any of the preceding clauses 5 to 6, wherein the percentages of (C) at each transition location provide a quantitative level of 5hmC or 5mC at each location of the nucleic acid.
8. The method according to any of the preceding clauses, wherein the nucleic acid sample comprises about 0,0001% of the whole genome of an organism.
9. The method according to any of the preceding clauses, wherein the first adapter-splinted oligonucleotide complex, the second adapter-splinted oligonucleotide complex are formed by combining a first adapted oligonucleotide (SEQ ID NO:1) with a first splinted oligonucleotide (SEQ ID NO:3) in the presence of T4 RNA Ligase Buffer and, separately combining a second adapted oligonucleotide (SEQ ID NO:2) with a second splinted oligonucleotide (SEQ ID NO:4) in the presence of a T4 RNA Ligase Buffer
10. The method according to any of the preceding clauses, wherein the nucleic acid sample results from an enrichment step before step a).
11. The method according to any of the preceding clauses, wherein the nucleic acid sample is fragmented into fragments of a sequence length between 100 and 1000 bp, preferably between 200 and 800 bp, more preferably between 250 and 700 bp, even more preferably around 500 bp.
12. The method according to any of the preceding clauses, wherein the nucleic acid sample is in the range of 1 pg to about 1000 µg DNA.
13. The method according to any of the preceding clauses, wherein the protecting group added to the 5-hydroxymethylcytosine (5hmC) is a glucose derived group
14. The method according to the preceding clauses, wherein the step of adding a protecting group to the 5- hydroxymethylcytosine (5hmC) in at least one of the nucleic acid samples comprises contacting said nucleic acid samples with an enzyme, preferably BGT enzyme.
15. The method according to any of the preceding clauses, wherein 5-methylcytosine (5mC) is oxidized into 5-carboxylcytosine (5caC) and/or 5-Formylcytosine by contacting the nucleic acid sample with an enzyme, preferably TET enzyme.
16. The method according to any of the preceding clauses, wherein the nucleic acid sample is denatured into ssNA at a temperature comprised between 70°C to 100°C in the presence of formamide.
17. The method according to any of the preceding clauses, wherein the enzymatic deamination is performed by one or more APOBEC enzymes selected from the group consisting of:
   human APOBEC: APOBEC3G, APOBEC3C, APOBEC3H, APOBEC3D, APOBEC3F, APOBEC3B, APOBEC3A, APOBEC2 and APOBEC1,
   mouse APOBEC: Apobec3, Apobec2, and Apobec1, and/or
   rat APOBEC: Apobec1
18. The method according to any of the preceding clauses, wherein the ssNA is combined with SSB between steps e1)/e2) and f1)/f2) of clause 1 and/or clause 3.
19. The method according to the preceding clause, wherein the SSB protein is selected from the group consisting of eukaryotic and prokaryotic SSB, preferably Extreme Thermostable SSB.
20. The method according to any of the preceding clauses, wherein in steps f1) and/or f2) the adjacent ends of the first adapter oligonucleotide and ssNA are covalently linked and the adjacent ends of the second adapter oligonucleotide and ssNA are covalently linked, the covalent linking of the adjacent ends is performed by enzymatic ligation.
21. The method according to any of the preceding clauses, wherein the conditions of step f1) and/or f2) are a temperature range between 25°C and 45 °C, preferably between 30°C and 38°C during a period of time between 30 minutes and 180 hours, preferably 40 min and 90 minutes.
22. The method according to any of the preceding clauses, wherein steps f1) and/or f2) comprises combining:
   a complex comprising the first splint oligonucleotide hybridized to the first adapter oligonucleotide via the first adapter oligonucleotide hybridization region,
   a complex comprising the second splint oligonucleotide hybridized to the second adapter oligonucleotide via the second adapter oligonucleotide hybridization region, and
   the ssNA.
23. The method according to any of the preceding clauses, wherein steps f1) and/or f2) comprises combining:
   a complex comprising the first splint oligonucleotide hybridized to the ssNA via the ssNA hybridization region,
   a complex comprising the second splint oligonucleotide hybridized to the ssNA via the ssNA hybridization region,
   the first adapter oligonucleotide, and
   the second adapter oligonucleotide.
24. The method according to any of the preceding clauses, wherein the blocking modification is selected from the group consisting of: the absence of a 3' OH at the end of the adapter oligonucleotide not adjacent to ssNA; and an inaccessible 3' OH at the end of the adapter oligonucleotide not adjacent to the ssNA.
25. The method according to the preceding clauses, wherein the blocking modification of the oligonucleotides is an inaccessible 3' OH at the end of the adapter oligonucleotide not adjacent to the ssNA, and wherein the blocking modification is selected from one or more of the group consisting of: an amino modifier, a spacer, a dideoxy base, an inverted dideoxy base, a 3' phosphate.
26. The method according to any of the preceding clauses, wherein the ssNA hybridization region comprises a random sequence.
27. The method according to any of the preceding clauses, wherein the ssNA hybridization region comprises a universal base.
28. The method according to any of the preceding clauses, wherein the adapter oligonucleotide comprises:
   an adapter for PCR amplification or a complement thereof; and/or a partial or complete sequencing adapter or a complement thereof.
29. The method according to any of the preceding clauses, wherein the method is performed without a temperature change between steps
   f1) and g1) and/or
   f2) and g2) and
   step g1) or g2) is started in less than 6hr after step f1) or f2) is finished.
30. The method according to any of the preceding clauses, wherein the method further comprises the step of amplifying the copy number of the dsDNA library after step g1) and/or g2) and before step h1) and/or h2).
31. A kit for performing the method described in any of the preceding clauses.

Unless defined otherwise, all technical and scientific terms used herein have the same meaning as commonly understood by one of ordinary skill in the art to which the methods, compositions and kits belong. Although any methods, compositions and kits similar or equivalent to those described herein can also be used in the practice or testing of the methods, compositions and kits, representative illustrative methods, compositions and kits are now described.

Each of the terms "comprising," "consisting essentially of," and "consisting of" may be replaced with either of the other two terms. The term "a" or "an" can refer to one of or a plurality of the elements it modifies (e.g., "a reagent" can mean one or more reagents) unless it is contextually clear either one of the elements or more than one of the elements is described. The term "about" as used herein refers to a value within 10% of the underlying parameter (i.e., plus or minus 10%; e.g., a weight of "about 100 grams" can include a weight between 90 grams and 110 grams). Use of the term "about" at the beginning of a listing of values modifies each of the values (e.g., "about 1, 2 and 3" refers to "about 1 , about 2 and about 3"). When a listing of values is described the listing includes all intermediate values and all fractional values thereof (e.g., the listing of values "80%, 85% or 90%" includes the intermediate value 86% and the fractional value 86.4%). When a listing of values is followed by the term "or more," the term "or more" applies to each of the values listed (e.g., the listing of "80%, 90%, 95%, or more" or "80%, 90%, 95% or more" or "80%, 90%, or 95% or more" refers to "80% or more, 90% or more, or 95% or more"). When a listing of values is described, the listing includes all ranges between any two of the values listed (e.g., the listing of "80%, 90% or 95%" includes ranges of "80% to 90%", "80% to 95%" and "90% to 95%"). Certain implementations of the technology are set forth in examples below

### Brief description of the figures

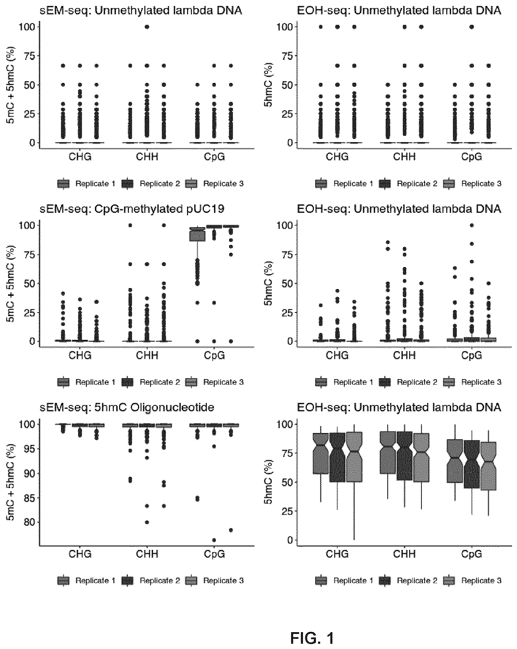

**Figure 1****.** Conversion rates of sEM-seq and EOH-seq using 3 controls (unmethylated λ DNA, CpG-methylated pUC19, 5hmC oligonucleotide), 3 replicates and 3 cytosine contexts (CHG, CHH and CpG). Y-axis show the conversion rate of 5mC + 5hmC (M-seq) or 5hmC (EOH-seq). Unmethylated A DNA are expected to show a minimal modification frequency in both experiments. CpG-methylated pUC19 control is only expected to show high modification frequency in sEM-seq when assessing the CpG context. The hydroxymethylated oligonucleotide is expected to show high frequencies of modification in both experiments.

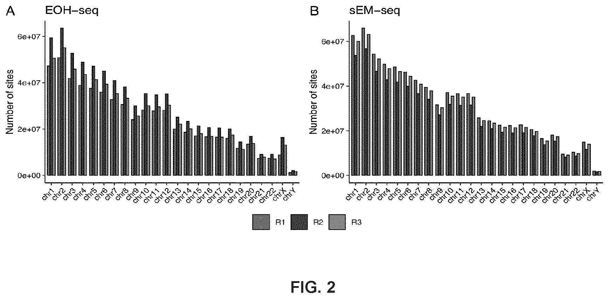

**Figure 2****.** Genome-wide distribution of informative C sites after (A) EOH-seq and (B) sEM-seq. The number of cytosines for which modifications can be detected is dependent on the chromosome length. Cytosines with coverage equal or higher than 5 are shown. Replicates 1, 2 and 3 are coloured in gray, red and yellow, respectively and shown in the following order: left-centre-right.

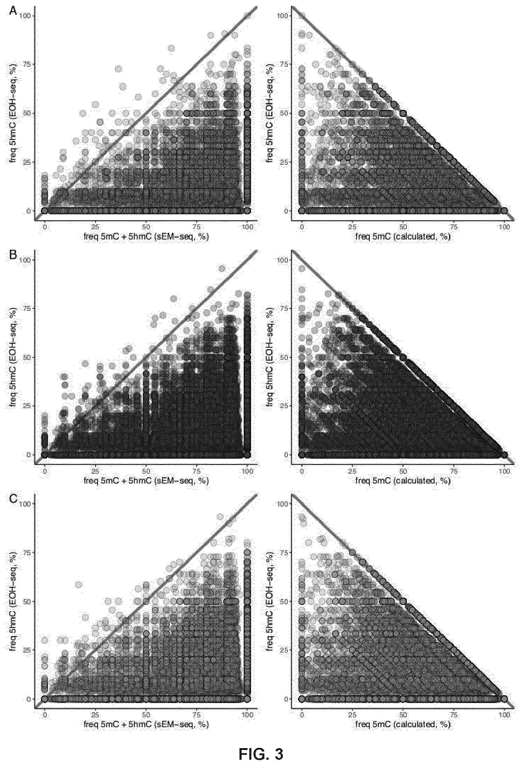

**Figure 3****.** 5hmC and 5mC frequency values for cytosines in CpG context across chromosome 21 in (A) Replicate 1, (B) Replicate 2 and (C) Replicate 3. Cytosines with coverage equal or higher than 10 are shown.

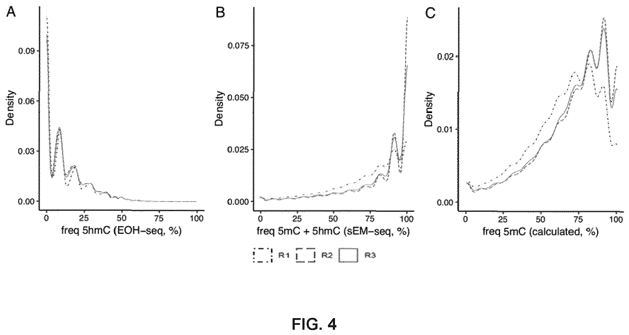

**Figure 4****.** Distribution of frequencies of cytosine modifications in CpG context across chromosome 21 in (A) EOH-seq, (B) sEM-seq and (C) of the calculated 5mC (subtracting the frequencies of EOH-seq and M-seq). Cytosines with coverage equal or higher than 10 are shown. Replicates 1, 2 and 3 are coloured in gray, red and yellow, respectively.

### Examples

### Materials and methods

### Nucleic acid samples and controls

Genomic DNA was extracted from human hippocampal brain tissue with a MagAttract HMW DNA Kit (Qiagen) according to the manufacturer's instructions. The resulting DNA extracts were quantified using the Qubit 2.0 Fluorometer, and DNA purity was assessed on a Nanodrop.

To assess the efficiency of the enzymatic conversion reaction of cytosine, 5-methylcytosine and 5-hydroxymethylcytosine we used three different spike-in controls.

Unmethylated lambda DNA and fully CpG methylated pUC19 DNA controls (E7123 & E7122) were obtained from the NEBNext^{®} Enzymatic Methyl-seq Conversion Module (New England Biolabs). As a 5hmC standard, a 879-bp synthetic dsDNA oligonucleotide with -50% G-C content and all Cs as 5hmC (D5405-3, Zymo Research) was used. Before each conversion experiment, 200 ng of genomic DNA spiked in with 0.02 ng of unmethylated lambda DNA, 0.003 ng of CpG methylated pUC19 DNA and 0.01 ng of synthetic 5hmC oligonucleotide were sonicated to a fragment size of approximately 300 bp (Q800R3, QSonica).

### Specific enzymatic deamination for 5hmC detection

The following conversion protocol is a modification of the NEBNext^{®} Enzymatic Methyl-seq Conversion Module (E7125, New England Biolabs). Before conversion, the fragmented DNA was cleaned up using homemade *solid-phase reversible immobilization* (SPRI) beads (beads/DNA ratio 2) to a final volume of 28 µL (eluted with H2O) (Rohland N, Reich D. Cost-effective, high-throughput DNA sequencing libraries for multiplexed target capture. Genome Res. 2012;22(5):939-946. doi:10.1101/gr.128124.111).

In order to glycosylate 5-hydroxymethylcytosine, purified DNA was mixed with 1 µL of Oxidation Enhancer (E7129-NEB), 1 µL of Oxidation Supplement (E7128-NEB), 1 µL of DTT (E7139-NEB) and 22 µL of nuclease-free water to a final volume of 50 µL, this mix was incubated at 37°C for 1h.

Glycosylated DNA was then incubated for 30 minutes with 1 µL of Stop Reagent (E7132-NEB) and purified with homemade SPRI beads (beads/DNA ratio 2) to a final volume of 16 µL (eluted with H2O). Purified DNA was then denatured by mixing it with 4 µL of formamide at 85°C for 10 minutes. Denatured DNA was then placed immediately on ice and deaminated by adding 68 µL of nuclease-free water, 10 µL of APOBEC Reaction Buffer (E7134), 1 µL of BSA (E7135) and 1 µL of APOBEC (E7133) to a total volume of 100uL and incubated at 37°C for 3 hours. Deaminated DNA was then purified using homemade SPRI beads (ratio 2) to a final volume of 24 µL (eluted with low EDTA TE buffer, pH 8.0) and used for library preparation.

### Enzymatic deamination for the detection of 5mC and 5hmC

The following conversion protocol is based on the NEBNext^{®} Enzymatic Methyl-seq Conversion Module (E7125, New England BioLabs). Before conversion, the fragmented DNA (sonicated as described above) was cleaned up using homemade SPRI beads (beads/DNA ratio 2) to a final volume of 28 µL (eluted with H2O). For the oxidation and glucosylation steps, purified DNA was mixed with 10 uL of TET2 Buffer (E7126-E7127), 1 µL of Oxidation Supplement (E7128), 1 µL of DTT (E7139), 1 µL of Oxidation Enhancer (E7129) and 4 µL of TET2 (E7130) to a final volume of 45 µL, then 5 µL of 400uM Fe(II) (diluted from E7131) solution was added and the resulting mix was incubated at 37°C for 1h. The converted DNA was then incubated for 30 minutes with 1 µL of Stop Reagent (E7132) and purified with homemade SPRI beads (beads/DNA ratio 2) to a final volume of 16 µL (eluted with H2O). Purified DNA was then denatured by adding 4 µL of formamide at 85°C for 10 minutes. Denatured DNA was then placed immediately on ice and deaminated by adding 68 µL of nuclease-free water, 10 µL of APOBEC Reaction Buffer (E7134), 1 µL of BSA (E7135) and 1 µL of APOBEC (E7133) to a total volume of 100 µL and incubated at 37°C for 3 hours. Deaminated DNA was then purified using homemade SPRI beads (ratio 2) to a final volume of 24 µL (eluted with low EDTA TE buffer, pH 8.0) and used for library preparation.

### Library preparation from converted and deaminated DNA

A minimum starting DNA amount of 10 ng resulting from either the specific enzymatic deamination for 5hmC detection or enzymatic deamination for the detection of 5mC and 5hmC were separately converted into Illumina sequencing libraries by following the Santa Cruz Reaction method (Kapp JD, Green RE, Shapiro, B. A Fast and Efficient Single-stranded Genomic Library Preparation Method Optimized for Ancient DNA. J Hered. 2021;112(3):241-249. doi:10.1093/jhered/esab012) with minor modifications:
Since the DNA after conversion is single-stranded, it must be quantified accordingly (eg. Qubit^{®} ssDNA Assay Kit).

In this particular example, the starting 10ng of DNA is combined with SSBs and heat denatured. After denaturation, the reactions were placed on ice or a PCR cooler at 4°C. After cooling, adapters were added to each reaction. Then, the reaction master mix is added, followed by mixing. Incubation at 37°C allows for adapter ligation to begin immediately with most ligation occurring before 45 minutes. Reactions can be cleaned up with established methods.

To prepare the adapter-splinted complexes, the first adapter oligonucleotide (SEQ ID NO:1) in a final concentration of 12 µM was combined with the first splint oligonucleotide (SEQ ID NO: 3) to a final concentration of 12 µM with 1 X final concentration of T4 RNA Ligase Buffer (Cat# B0216L). A similar, separate mixture was prepared using the second adapter oligonucleotide (SEQ ID NO:2) and the second splint oligonucleotide (SEQ ID NO: 4). The oligos were hybridized by heating to 95°C for 10 seconds and then and then ramped down to 10°C at a rate of 0.1°C/s. Then, first adapter-splinted complex and the second adapter-splinted complex were diluted to 0,75 µM and 0,375 µM respectively. The second adapter-splinted complex binds more easily to the template, and thus also reduces adapter artefacts, that is why less concentration is used.

After the ligation of splinted adapters to the deaminated ssNA, samples were immediately subjected (in less than 3hr) to the indexing PCR. The complexes comprising deaminated ssNA with adapter and splinted oligonucleotides were not subjected to big temperature changes before continuing with the indexing PCR.

An example protocol is provided below.
1. Sample input: Combine 10 ng of converted DNA with 41 ng ET SSB (Cat# M2401 S) to a final volume of 22ul
2. Denature sample:
   a. Incubate samples in a thermocycler, with a pre-heated lid, at 95°C for 3 minutes
   b. Immediately place denatured sample on ice or a PCR cooler for 2 minutes.
3. Add 1µl of each splinted adapter dilution (equal volume of first adapter-splinted complex and the second adapter-splinted complex)
   a. Adapter input will depend on the molarity of the input. A molar ratio between 6 and 10 to 1, second adapter-splinted complex to template, is preferred and 10 to 20, first adapter-splinted complex to template.
4. Add reaction master mix and mix thoroughly by pipetting
   a. 3,8 µl of SCR buffer
   b. 19,3 µl of 50% PEG 8000 (NEB, Cat# B0216L)
   c. 1 µl of 10,000 U/ml T4 Polynucleotide Kinase (Thermo, Cat# M0201 L)
   d. 1 µl of 2,000,000 U/ml T4 DNA Ligase (Thermo, Cat# M0202M)
   e. 0,5 µl of 1M DTT (Thermo, Cat# P2325)
   f. 0,5 µl of 100mM ATP (Thermo, Cat# R0441)
5. Incubate at 37°C for 45 minutes
   a. Most ligation occurs in the first 15 minutes but the plateau is not achieved until around 45 minutes.

Each library was amplified by PCR with Q5U Master Mix (M0597, New England Biolabs) using two uniquely barcoded primers. After index PCR, libraries were purified with homemade SPRI beads (ratio 1) and eluted in 25 µL of low EDTA TE buffer (pH 8.0). Libraries were quantified using a BioAnalyzer and sequenced on a Nextseq 500 (Illumina) with 2x150 reads, using regular protocols in the field that a skilled person would know.

### Results

### Estimating the performance of the methods of the invention in positive controls

In order to demonstrate the advantages of the method of the invention we used three nucleic acid samples as DNA controls:
1. Unmethylated A DNA (48,502 bp)
2. CpG-methylated pUC19 (2,686 bp)
3. Oligonucleotide 5hmC (879 bp)

In Control 1 (Unmethylated A DNA), all (C) are unmodified, so they will appear as (T) in EOH-seq, M-seq and sEM-seq.

In Control 2 (CpG-methylated pUC19), only (C) in a CpG context are methylated (5mC). They should be seen as (T) in EOH-seq and as (C) in sEM-seq. All (C) in non-CpG contexts should be seen as unmethylated, thus (T), so they can be used as negative controls in both methods.

Control 3 (oligonucleotide 5hmC) has all C hydroxymethylated (5hmC), so they should be seen as (C) in EOH-seq, M-seq and sEM-seq.

We added these controls in an experiment with human DNA from a hippocampus sample. To assess the variability of the protocol, we performed three replicates in each of the experiments. The sequencing data was analyzed using standard methylation pipelines (Krueger F, Andrews SR. Bismark: a flexible aligner and methylation caller for Bisulfite-Seq applications. Bioinformatics. 2011;27(11):1571-1572. doi:10.1093/bioinformatics/btr167). Figure 1 shows the performance of the three controls in the two experiments and in three different cytosine contexts: H is any nucleotide except G. For CHG it is any of the combinations CAG, CCG, CTG. The same for CHH but even more combinations. The aim of this variation is whether the method of the invention goes better or not depending on the context in which the methylation is.

### Detection of methylation and/or hydroxymethylation patterns across the human genome

An innovation of the method of the invention is that it allows for the accurate detection of 5hmC modifications across the genome. Moreover, in M-seq by subtracting the values found in step (2) by those in step (1), it is also possible to specifically determine the 5mC values genome-wide. We applied this methodology to determine both modifications in genomic DNA from a human hippocampal brain tissue from a male individual, in which we performed three replicates of each experiment.

Figure 2 shows the number of informative sites with methylation and/or hydroxymethylation for each chromosome and replicate in both experiments. Since sEM-seq provides information about 5mC and 5hmC and EOH-seq is able to report the specific frequencies of 5hmC (Figure 3, left panels), by subtracting the frequencies of both experiments, the real 5mC levels can be known (Figure 3, right panels). The resulting distribution of the frequencies is the expected for both modifications (Figure 4).

### Conclusions

The method of the present invention specifically identifies 5-hydroxymethylcytosine (5hmC) (EOH-seq) in a nucleic acid sample of the whole genome with a conversion rate of around 80% and specificity close to 100%. Moreover, the method of the invention is able to specifically detect and discriminate 5-methylcytosine (5mC) from (5hmC) in a nucleic acid sample.

### Sequences listing

SEQ ID NO: 1 first adapter oligonucleotide sequence (5'-3'):
   ACACTCTTTCCCTACACGACGCTCTTCCGATCT
SEQ ID NO: 2 second adapter oligonucleotide sequence (5'-3'):
   AGATCGGAAGAGCACACGTCTGAACTCCAGTCAC
SEQ ID NO: 3 first splint oligonucleotide sequence (5'-3'):
   NNNNNNNAGATCGGAAGAGCGTCGTGTAGGGAAAGAGTGT
SEQ ID NO: 4 second splint oligonucleotide sequence (5'-3'):
   GTGACTGGAGTTCAGACGTGTGCTCTTCCGATCTNNNNNNN
SEQ ID NO: 5 P5 Index primer (5'-3'): 
AATGATACGGCGACCACCGAGATCTACACNNNNNNNACACTCTTTCCCTACACGA
CGCTCTT
SEQ ID NO: 6 P7 Index primer (5'-3'):
   CAAGCAGAAGACGGCATACGAGATNNNNNNNGTGACTGGAGTTCAGACGTGT

## Claims

1. A method to detect 5-hydroxymethylcytosine (5hmC) in a nucleic acid sample, comprising the following steps:
**a)** providing a nucleic acid sample, said nucleic acid sample being
a genomic DNA sample or
a genomic DNA sample that has gone through a previous enrichment step for a target region of the genome,
and fragmenting said nucleic acid sample into fragments of a sequence length between 100 base pairs and 10000 base pairs,
**b)** adding a protecting group to the 5-hydroxymethylcytosine (5hmC) in the fragmented nucleic acid sample of step a),
**d1)** denaturing the product of step b) to obtain a modified single stranded nucleic acid sample (ssNA),
**e1)** performing enzymatic deamination of the ssNA product obtained in step d1) to convert cytosines (C) into uracils (U), and performing cleanup of said deaminated ssNA
**f1)** combining the products of step (e) with
a first adapter-splinted oligonucleotide complex, and
a second adapter-splinted oligonucleotide complex
by enzymatic ligation wherein the first and second adapter-splinted oligonucleotide complex hybridize to the ssNA via a ssNA hybridization region,
**g1)** amplifying by indexing PCR the products of step f1) by combining said products with at least two different sequencing adapters, to obtain a dsDNA nucleic acid library and
**h1)** sequencing the nucleic acid sample contained in the dsDNA nucleic acid library of step g1),
wherein the presence of a cytosine (C) in the same position of the dsDNA nucleic acid library and in the original nucleic acid sequence of step a) provides the location of 5hmC in a nucleic acid sample.

2. A method to detect 5-methylcytosine (5mC) and 5-hydroxymethylcytosine (5hmC) in a nucleic acid sample comprising:
**a)** providing a nucleic acid sample, said nucleic acid sample being
a genomic DNA sample or
a genomic DNA sample that has gone through a previous enrichment step for a target region of the genome,
and fragmenting said nucleic acid sample into fragments of a sequence length between 100 base pairs and 10000 base pairs,
**c)** adding a protecting group to the 5-hydroxymethylcytosine (5hmC) and oxidizing the 5-methylcytosine (5mC) of said fragmented second nucleic acid sample
**d2)** denaturing the product of step c) to obtain a modified single stranded nucleic acid sample (ssNA),
**e2)** performing enzymatic deamination of the ssNA product obtained in step d2) to convert cytosines (C) into uracyls (U), and performing cleanup of said deaminated ssNA
**f2)** combining the products of step (e2) with
a first adapter-splinted oligonucleotide complex, and
a second adapter-splinted oligonucleotide complex
by enzymatic ligation wherein the first and second adapter-splinted oligonucleotide complex hybridize to the ssNA via a ssNA hybridization region,
**g2)** amplifying by indexing PCR the products of step f2) by combining said products with at least two different sequencing adapters, to obtain a dsDNA nucleic acid library and
**h2)** sequencing the nucleic acid sample contained in the dsDNA nucleic acid library of step g2),
wherein the presence of a cytosine (C) in the same position of the dsDNA nucleic acid library and in the original nucleic acid sequence of step a) provides the location of 5hmC or 5mC in a nucleic acid sample.

3. A method to detect and to discriminate 5-methylcytosine (5mC) from 5-hydroxymethylcytosine (5hmC) in a nucleic acid sample comprising:
providing a nucleic acid sample, said nucleic acid sample being
a genomic DNA sample or
a genomic DNA sample that has gone through a previous enrichment step for a target region of the genome,
fragmenting said nucleic acid sample into fragments of a sequence length between 100 base pairs and 10000 base pairs,
dividing it into at least a first nucleic acid sample and a second nucleic acid sample
**1)** subjecting said fragmented first nucleic acid sample to steps b) to h1) of the method of claim 1,
**2)** subjecting said fragmented second nucleic acid sample to steps c) to h2) of the method of claim 2,
**3)** comparing the results of steps (1), (2), wherein the presence of a cytosine (C) in the sequence of the dsDNA nucleic acid library of either (1) and/or (2), and/or in the original nucleic acid sequence of step a) provides the location and discrimination of 5-methylcytosine (5mC) from 5-hydroxymethylcytosine (5hmC).

4. The method according to any of the preceding claims, wherein the first adapter-splinted oligonucleotide complex and the second adapter-splinted oligonucleotide complex are formed by combining a first adapted oligonucleotide (SEQ ID NO:1) with a first splinted oligonucleotide (SEQ ID NO:3) in the presence of T4 RNA Ligase Buffer and, separately combining a second adapted oligonucleotide (SEQ ID NO:2) with a second splinted oligonucleotide (SEQ ID NO:4) in the presence of a T4 RNA Ligase Buffer

5. The method according to the preceding claim, wherein the step of adding a protecting group to the 5- hydroxymethylcytosine (5hmC) in at least one of the nucleic acid samples comprises contacting said nucleic acid samples with an enzyme, preferably BGT enzyme.

6. The method according to any of the preceding claims, wherein 5-methylcytosine (5mC) is oxidized into 5-carboxylcytosine (5caC) and/or 5-Formylcytosine by contacting the nucleic acid sample with an enzyme, preferably TET enzyme.

7. The method according to any of the preceding claims, wherein the nucleic acid sample is denatured into ssNA at a temperature comprised between 70°C to 100°C in the presence of formamide.

8. The method according to any of the preceding claims, wherein the enzymatic deamination is performed by one or more APOBEC enzymes selected from the group consisting of:
human APOBEC: APOBEC3G, APOBEC3C, APOBEC3H, APOBEC3D, APOBEC3F, APOBEC3B, APOBEC3A, APOBEC2 and APOBEC1,
mouse APOBEC: Apobec3, Apobec2, and Apobec1, and/or
rat APOBEC: Apobec1

9. The method according to any of the preceding claims, wherein the ssNA is combined with SSB between steps e1)/e2) and f1)/f2) respectively.

10. The method according to any of the preceding claims, wherein in steps f1) and/or f2) the adjacent ends of the first adapter oligonucleotide and ssNA are covalently linked and the adjacent ends of the second adapter oligonucleotide and ssNA are covalently linked, the covalent linking of the adjacent ends is performed by enzymatic ligation.

11. The method according to any of the preceding claims, wherein the conditions of step f1) and/or f2) are a temperature range between 25°C and 45 °C, preferably between 30°C and 38°C during a period of time between 30 minutes and 180 hours, preferably 40 min and 90 minutes.

12. The method according to any of the preceding claims, wherein the blocking modification is selected from the group consisting of: the absence of a 3' OH at the end of the adapter oligonucleotide not adjacent to ssNA; and an inaccessible 3' OH at the end of the adapter oligonucleotide not adjacent to the ssNA.

13. The method according to the preceding claim, wherein the blocking modification of the oligonucleotides is an inaccessible 3' OH at the end of the adapter oligonucleotide not adjacent to the ssNA, and wherein the blocking modification is selected from one or more of the group consisting of: an amino modifier, a spacer, a dideoxy base, an inverted dideoxy base, a 3' phosphate.

14. The method according to any of the preceding claims, wherein the method is performed without a temperature change between steps
f1) and g1) and/or
f2) and g2) and
step g1) or g2) is started in less than 6hr after step f1) or f2) is finished.

15. A kit for performing the method described in any of the preceding claims.
